# EUROPÄISCHE PATENTSCHRIFT

(11) **EP 2 257 649 B1**
(45) Veröffentlichungstag und Bekanntmachung des Hinweises auf die Patenterteilung: **09.11.2011**
(21) Anmeldenummer: 09720794.8
(22) Anmeldetag: 09.03.2009
(51) Int. Cl.: C14C 3/06, C14C 9/00

(54) **VERFAHREN ZUR HERSTELLUNG VON LEDER MIT GERINGER NEIGUNG ZUM VERGILBEN**
METHOD FOR THE PRODUCTION OF LEATHER HAVING A LOW TENDENCY TO DISCOLOR
PROCÉDÉ DE PRODUCTION DE CUIR À LÉGÈRE TENDANCE AU JAUNISSEMENT

(30) Priorität: 13.03.2008 EP 08152701
(43) Veröffentlichungstag der Anmeldung: 08.12.2010
(60) Teilanmeldung: 11163456.4
(73) Patentinhaber: BASF SE, 67056 Ludwigshafen (DE)
(72) Erfinder: HÜFFER, Stephan, 67063 Ludwigshafen (DE); GARNIER, Sebastien, 69469 Weinheim (DE); BETTE, Virginie, 68165 Mannheim (DE); BOHRES, Edward, 68161 Mannheim (DE); WOLF, Gerhard, 68775 Ketsch (DE); ANNEN, Ulrich, 67454 Haßloch (DE); GLOCKNITZER, Franz, 67480 Edenkoben (DE)
(86) Internationale Anmeldenummer: PCT/EP2009/052719
(87) Internationale Veröffentlichungsnummer: WO 2009/112453

(56) Entgegenhaltungen:
- WO-A-2005/023886
- WO-A-2007/063047
- DE-A1- 19 860 610
- US-A1- 2007 027 243

## Beschreibung

Die vorliegende Erfindung betrifft ein Verfahren zur Herstellung von Leder, dadurch gekennzeichnet, dass man Cr(III)-haltige Halbzeuge in wässrigem Milieu mit einem oder mehreren Thioethern mit mindestens 10 C-Atomen pro Molekül behandelt.

Weiterhin betrifft die vorliegende Erfindung Leder und ihre Verwendung zur Herstellung von Schuhen, Möbeln und Automobilinnenteilen.

Die Herstellung von Ledern mit geringer Neigung zum Vergilben, auch nach längerer Verwendungszeit, ist eine Aufgabe, die immer mehr Aufmerksamkeit genießt. Vergilbende Leder sehen nicht nur unästhetisch aus, auch ihre mechanischen Eigenschaften lassen in der Regel stark nach. Für die Vergilbungsneigung werden verschiedene Lederhilfsmittel verantwortlich gemacht, beispielsweise die Gerbmittel. So ist bekannt, dass viele Polykondensatgerbstoffe die Vergilbung von Leder nicht vermindern können.

Auch die Wahl der Fettungsmittel kann die Vergilbungsneigung beeinflussen.

Die Herstellung von Cr(V1)-armen Ledern ist eine Herausforderung, der immer mehr Aufmerksamkeit geschenkt wird. Spuren von Cr(V1) können in Ledern entstehen, die mit Hilfe von Cr(III)-Verbindungen wie beispielsweise Cr₂(SO₄)₃·n H₂O gegerbt wurden. Aus Umwelt- und Gesundheitsgründen ist die Entstehung von Cr(VI) in Leder unerwünscht. Es hat bereits verschiedene Versuche gegeben, die Bildung von Cr(VI) in Leder zu vermeiden.

So gibt es zahlreiche Versuche, durch chromfreie Gerbung den Gehalt an Cr(III) im Leder und damit auch die Bildung von Cr(VI) vollkommen zu vermeiden. Derartige Leder lassen sich auch in einigen Fällen einsetzen. Chromfrei gegerbte Halbzeuge sind auch als "wet white" bekannt. Jedoch sind chromfrei gegerbte Leder den Ledern auf Basis von wet blue in vielen Fällen noch unterlegen, was die mechanischen Eigenschaften betrifft. Außerdem können viele Leder auf Basis von wet white mit Ledern auf Basis von Wet blue nicht mithalten, was die Langzeitvergilbung betrifft, so dass noch immer mehr als 85% der Leder weltweit auf Basis von chromhaltigen Gerbstoffen hergestellt werden.

Es wurde vorgeschlagen, L-Ascorbinsäure oder D-Isoascorbinsäure nach der Chromgerbung zuzusetzen, s. WO 2007/063047. Die Wirkung von L-Ascorbinsäure und D-Isoascorbinsäure ist jedoch nicht langfristig. Außerdem kann L-Ascorbinsäure die Analytik von Cr(VI) beeinflussen, indem Ascorbinsäure als Ligand für Cr(VI) wirkt.

Es wurde weiterhin vorgeschlagen, Ethylendiamintetraessigsäure (EDTA) bei der Chromgerbung zuzusetzen. Jedoch kann auch EDTA Cr(VI) maskieren. Außerdem sind EDTA-haltige Abwässer unerwünscht, weil EDTA Schwermetalle aus Schlämmen mobilisieren kann.

DE 198 60 610 schlägt vor, bei der Fettung ein oder mehrere Antioxidantien zuzusetzen, beispielsweise Ascorbinsäure, Bisphenolderivate oder Di-n-Butyldithiocarbamate (Seite 2, Zeile 55). Die Langzeitwirkung derartiger Verbindungen ist jedoch für manche Zwecke noch nicht gut genug, insbesondere lässt in Langzeittests die Wärmevergilbung noch zu wünschen übrig.

DE 100 28 142 schlägt ebenfalls vor, Antioxidantien während des Pickelns oder während der Gerbung mit Cr(III)-Verbindungen einzusetzen, und nennt sterisch gehinderte Phenole wie 4,4'-Methylenbis-(2,6-di-tert.-butylphenol). Auch hier lässt sich die Langzeitwirkung noch verbessern.

Es wurde weiterhin vorgeschlagen, Gallussäure bei der Vor- oder Nachgerbung einzusetzen. Problematisch ist jedoch, dass Gallussäure zusammen mit Eisensalzen tiefschwarze Tinten geben kann. Eisenverbindungen sind in der Gerbung stets zugegen, beispielsweise aufgrund der Abnutzung der Spaltmaschinen. Man erhält beim Einsatz von Gallussäuren in der Gerbung oder Nachgerbung also schwarze oder dunkelgraue Leder, die sich nicht zur Herstellung von beispielsweise weißen Schuhen oder Möbelteilen eignen.

Es bestand also die Aufgabe, ein Verfahren zur Herstellung von Leder bereit zu stellen, das auch über einen längeren Zeitraum einen geringen Gehalt von Cr(VI) und außerdem über einen längeren Zeitraum sehr gute mechanische Eigenschaften bei gleichzeitig geringer Neigung zur Vergilbung aufweist. Weiterhin bestand die Aufgabe, Leder bereit zu stellen, die auch über einen längeren Zeitraum einen geringen Gehalt von Cr(VI) und außerdem über einen längeren Zeitraum sehr gute mechanische Eigenschaften bei gleichzeitig geringer Neigung zur Vergilbung aufweisen. Weiterhin bestand die Aufgabe, Schuhe, Möbel und Automobilinnenteile bereit zu stellen, die auch über einen längeren Zeitraum einen geringen Gehalt von Cr(VI) und außerdem über einen längeren Zeitraum sehr gute mechanische Eigenschaften bei gleichzeitig geringer Neigung zur Vergilbung aufweisen.

Dementsprechend wurde das eingangs definierte Verfahren gefunden.

Unter Cr(III)-haltigen Halbzeugen werden im Rahmen der vorliegenden Erfindung Zwischenprodukte in der Lederherstellung verstanden, die auf einer vorbehandelten Tierhaut, beispielsweise einer Haut von einem Säugetier wie Rind, Kalb, Schwein, Wild, Ziege oder Antilope, einem Fisch wie beispielsweise Aal, einem Vogel wie beispielsweise einem Strauß oder einem Reptil wie beispielsweise einer Schlange, basieren und die neben einigen Vorbehandlungsschritten auch mindestens einem Behandlungsschritt mit einer Cr(III)-Verbindung unterworfen worden sind, bei denen Cr(III)-Verbindungen in die Tierhaut eingelagert werden. Beispiele für derartige Behandlungsschritte mit einer Cr(III)-Verbindung sind Vorgerbung, Gerbung oder auch ein Pickel mit mindestens einer Cr(III)-Verbindung. Dabei ist es nicht erforderlich, dass Cr(III)-Verbindung in dem betreffenden Behandlungsschritt die einzige gerbend wirkende Verbindung ist, es kann sich auch um einen Behandlungsschritt mit einem Kombinationsgerbstoff oder einer Kombination von Gerbstoffen wie beispielsweise einer Mischung aus Cr(III)-Verbindung und einem Syntan, einem Polymergerbstoff oder einem Aldehyd wie beispielsweise Glutardialdehyd handeln.

Das eingangs definierte Verfahren geht aus von Cr(III)-haltigen Halbzeugen. Unter Cr(III)-haltig wird verstanden, dass die betreffenden Halbzeuge beispielsweise 0,01 bis 4 Gew.-%, bevorzugt 0,1 bis 2,5 Gew.-% Cr₂O₃·aq enthalten können, bezogen auf das Spaltgewicht und berechnet auf den Gehalt an Cr. Unter Cr(III)-Verbindungen, mit denen man Tierhaut behandeln kann, sind beispielsweise Cr₂(SO₄)₃, basische Cr-Sulfate, Chromalaune wie KCr(SO₄)₂·12 H₂O und Komplexverbindungen des dreiwertigen Chroms zu nennen.

Erfindungsgemäß behandelt man in wässrigem Milieu. Es ist möglich, Cr(III)-haltiges Halbzeug mit einer wässrigen Lösung oder Paste zu behandeln, die oxidierbare Verbindung enthält, die ausgewählt wird aus Thioethern mit mindestens 10 C-Atomen und Verbindungen der allgemeinen Formel III.

Vorzugsweise geht man so vor, dass man mit einer wässrigen Flotte behandelt, die mindestens einen Thioether mit mindestens 10 C-Atomen pro Molekül enthält. Thioether mit mindestens 10 C-Atomen pro Molekül werden im Rahmen der vorliegenden Erfindungen auch kurz als "Thioether mit mindestens 10 C-Atomen", als "eingesetzter Thioether" oder als "Thioether" bezeichnet.

In einer Ausführungsform der vorliegenden Erfindung weist eingesetzter Thioether einen Oktanol-Wasser-Koeffizient (Oktanol-Wasser-Verteilungskoeffizient) größer als 1 bzw. einen log pow größer null, bevorzugt einen Oktanol-Wasser-Koeffizient größer als 5 auf.

In einer Ausführungsform der vorliegenden Erfindung weist eingesetzter Thioether in destilliertem Wasser bei 20°C eine Löslichkeit von weniger als 10 mg/l, bevorzugt 0,01 bis 5 mg/l auf.

In einer Ausführungsform werden Thioether gewählt aus Verbindungen der allgemeinen Formel I 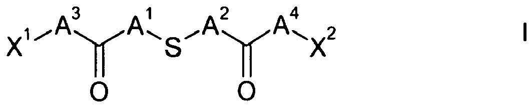 wobei die Variablen wie folgt definiert sind:
X¹ und X² sind verschieden oder vorzugsweise gleich und gewählt sind aus C₁₀-C₄₀-Alkyl, verzweigt oder vorzugsweise unverzweigt, beispielsweise n-Decyl, iso-Decyl, n-Dodecyl, iso-Dodecyl, n-Tetradecyl, n-Hexadecyl, n-Octadecyl, n-Eicosyl,
C₁₀-C₄₀-Alkoxy, verzweigt oder vorzugsweise unverzweigt und gewählt aus n-Decoxy, iso-Decoxy, n-Dodecoxy, iso-Dodecoxy, n-Tetradecoxy, n-Hexadecoxy, n-Octadecoxy, n-Eicosoxy,
C₆-C₁₄-Aryl, beispielsweise Phenyl, 1-Naphthyl, 2-Naphthyl, 1-Anthracenyl, 2-Anthraccenyl oder 9-Anthracenyl,
C₆-C₁₄-Hydroxyaryl, beispielsweise 2-Hydroxyphenyl, 3-Hydroxyphenyl, para-Hydroxyphenyl,
jeweils unsubstituiert oder vorzugsweise ein- oder mehrfach substituiert mit C₁-C₄-Alkyl, wobei C₁-C₄-Alkyl gewählt wird aus Methyl, Ethyl, n-Propyl, iso-Propyl, n-Butyl, iso-Butyl, sec.-Butyl und tert.-Butyl, wobei Methyl, iso-Propyl und tert.-Butyl bevorzugt sind,
und stickstoffhaltigen Heterocyclen, beispielsweise Pyridyl und insbesondere Isocyanurat,
A¹, A², A³ und A⁴ sind verschieden oder gleich und gewählt aus Spacern der Formel (CHR¹)ₙ, wobei R¹ gleich oder verschieden sein kann und unabhängig voneinander gewählt wird aus C₁-C₄-Alkyl, wobei C₁-C₄-Alkyl gewählt wird aus Methyl, Ethyl, n-Propyl, iso-Propyl, n-Butyl, iso-Butyl, sec.-Butyl und tert.-Butyl, wobei Methyl bevorzugt ist, oder insbesondere Wasserstoff,
und wobei n eine ganze Zahl im Bereich von null bis 10, bevorzugt im Bereich von eins bis vier und besonders bevorzugt zwei oder drei ist.

Bevorzugt sind X¹ und X², A¹ und A² einerseits und A³ und A⁴ andererseits jeweils paarweise gleich.

Beispiele für besonders bevorzugte substituierte C₆-C₁₄-Hydroxyarylgruppen sind 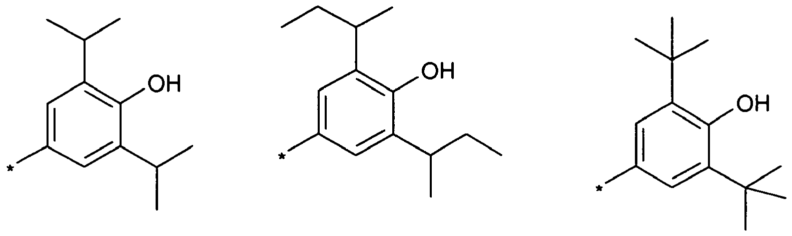 wobei jeweils die Bindung zu A³ bzw. A⁴ mit * gekennzeichnet ist.

Besonders bevorzugt sind X¹ und X², A¹ und A² einerseits und A³ und A⁴ andererseits jeweils paarweise gleich, und X¹ und X² sind jeweils gewählt aus n-Decoxy, n-Dodecoxy, n-Tetradecoxy, n-Hexadecoxy und n-Octadecoxy.

In einer Ausführungsform der vorliegenden Erfindung handelt sich bei Thioethern um solche, die mindestens einen sterisch anspruchsvoll substituierten Phenolrest tragen. Dabei werden unter sterisch anspruchsvoll substituierten Phenolresten solche verstanden, die mindestens einen sekundären oder tertiären Alkylsubstituenten in ortho-Stellung zur phenolischen OH-Gruppe tragen. Geeignete sekundäre und tertiäre Alkylsubstituenten sind beispielsweise iso-Propyl, sek.-Butyl, sek.-Amyl, tert.-Amyl und insbesondere tert.-Butyl.

In einer Ausführungsform können sterisch anspruchsvoll substituierte Phenolreste je einen sekundären oder tertiären Alkylrest in den beiden ortho-Stellungen zur Hydroxylgruppe tragen und einen weiteren Rest, beispielsweise einen linearen C₁-C₄-Alkylrest in meta- oder para-Stellung zur Hydroxylgruppe.

In einer bevorzugten Ausführungsform der vorliegenden Erfindung handelt es sich bei Thioethern um solche der der allgemeinen Formel II 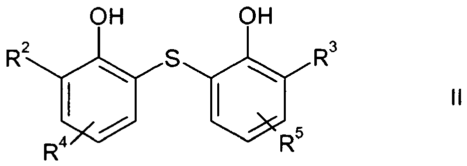 wobei die Variablen wie folgt definiert sind:
- R², R³: verschieden oder vorzugsweise gleich und gewählt aus iso-Amyl, tert.Amyl, iso-Propyl, tert.-Butyl und sec.-Butyl, insbesondere tert.-Butyl,
- R⁴, R⁵: verschieden oder vorzugsweise gleich und gewählt aus Phenyl, Benzyl und C₁-C₄-Alkyl, wobei C₁-C₄-Alkyl gewählt wird aus Methyl, Ethyl, n-Propyl, iso- Propyl, n-Butyl, iso-Butyl, sec.-Butyl und tert.-Butyl, vorzugsweise lineares C₁-C₄-Alkyl ,wobei Methyl bevorzugt ist,
und insbesondere Wasserstoff.

In einer Ausführungsform der vorliegenden Erfindung setzt man 0,005 bis 0,2 Gew.-% Thioether ein, bevorzugt 0,01 bis 0, 1 % bezogen auf das Falzgewicht des betreffenden Cr(III)-haltigen Halbzeugs.

Vorstehend genannte Thioether sind nur wenig wasserlöslich. Bevorzugt führt man das erfindungsgemäße Verfahren daher durch, indem man den oder die eingesetzten Thioether in Glycerin oder einem oder mehreren Oligomeren von Ethylenglykol solubilisiert oder löst oder aufschlämmt, bevorzugt sind Triethylenglykol und Glycerin, und dann im erfindungsgemäßen Verfahren einsetzt.

Unter Oligomeren von Ethylenglykol sind bei Zimmertemperatur flüssige Polymere des Ethylenglykols zu verstehen, beispielsweise Diethylenglykol, Tetraethylenglykol, Pentaethylenglykol und insbesondere Triethylenglykol.

In einer Ausführungsform der vorliegenden Erfindung behandelt man Halbzeuge vor, nach oder vorzugsweise gleichzeitig der Behandlung mit Thioether mit einem oder mehreren Fettungsmitteln, wobei das oder die Fettungsmittel natürlichen oder synthetischen Ursprungs sein können. Beispiele für Fettungsmittel sind natürliche oder vorzugsweise synthetische Wachse, native oder synthetische Öle oder natives oder synthetisches Fett. Bevorzugte Fettungsmittel sind native Fette und natürliche Öle wie z.B. Fischöle, insbesondere solche mit einem vergleichsweise hohen Anteil an ungesättigten Fettsäuren.

Als Beispiele für natürliche Wachse seien Bienenwachs, Korkwachs, Montanwachse oder Carnaubawachs genannt.

Als Beispiele für synthetische Wachse seien Polyethylenwachse oder Ethylencopolymerwachse genannt, wie sie beispielsweise durch radikalische Polymerisation von Ethylen oder radikalische Copolymerisation von Ethylen mit beispielsweise (Meth)acrylsäure oder durch Ziegler-Natta-Katalyse erhältlich sind. Weiterhin seien Polyisobutylenwachse genannt. Weiterhin seien Paraffingemische genannt; darunter sind Gemische von Kohlenwasserstoffen zu verstehen, die 12 oder mehr Kohlenstoffatome aufweisen und üblicherweise einen Schmelzpunkt im Bereich von 25 bis 45 °C aufweisen. Derartige Paraffingemische können beispielsweise in Raffinerien oder Crackern anfallen und sind dem Fachmann als Paraffingatsch und Sasolwachse bekannt. Ein weiteres Beispiel für synthetische Wachse sind Montanesterwachse.

Als Beispiele für natürliche Öle seien bei Zimmertemperatur flüssige Triglyceride genannt, beispielsweise Fischöl, Rinderklauenöl, Olivenöl, Baumwollsamenöl, Rizinusöl, Sonnenblumenöl und Erdnussöl genannt.

Als Beispiele für synthetische Öle seien Weißöl, Paraffinöl, funktionalisierte Paraffine wie beispielsweise chlorierte oder sulfochlorierte Paraffine oder auch Polyalkylenglykole wie beispielsweise Polyethylenglykol genannt.

Als Beispiele für natürliche Fette seien bei Zimmertemperatur feste native Triglyceride genannt wie beispielsweise Lanolin, Schellackwachs sowie deren Gemische.

Weitere geeignete Fettungsmittel sind sulfitierte oder sulfonierte natürliche Fette oder Öle, beispielsweise sulfitiertes Fischöl oder sulfoniertes Rinderklauenöl.

Die fettende Wirkung kann durch Zusatz von anionischem Emulgator noch verstärkt werden, beispielsweise C₁₀-C₄₀-Alkysulfonaten, C₁₀-C₄₀-Alkysulfaten oder Sulfobernsteinsäureestern der allgemeinen Formel IV 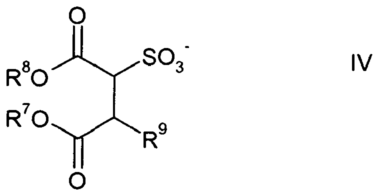 in denen die Variablen wie folgt definiert sind:
- R⁷, R⁸: gleich oder vorzugsweise verschieden und gewählt aus Wasserstoff, C₁-C₃₀-Alkyl, verzweigt oder unverzweigt, wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, iso-Amyl, n-Hexyl, iso-Hexyl, sec.-Hexyl, n-Heptyl, iso-Heptyl, n-Octyl, n-Nonyl, n-Decyl, n-Undecyl, n-Dodecyl, n-Tridecyl, n-Tetradecyl, n-Hexadecyl, n-Octadecyl, n-Eicosyl, bevorzugt in β-Stellung verzweigte Reste der Formel IV a

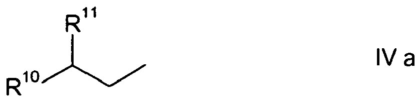

(CH₂CH₂O)ₓ-O-R¹² oder [CH(CH₃)CH₂O)ₓ-O-R¹², wobei x eine ganze Zahl im Bereich von 1 bis 20 ist,
C₆-C₁₄-Aryl, beispielsweise Phenyl, 1-Naphthyl, 2-Naphthyl, 1-Anthryl, 2-Anthryl, 9-Anthryl, 1-Phenanthryl, 2-Phenanthryl, 3-Phenanthryl, 4-Phenanthryl und 9-Phenanthryl, bevorzugt Phenyl, 1-Naphthyl und 2-Naphthyl, besonders bevorzugt Phenyl;
- R⁹: ist gewählt aus C₁-C₄-Alkyl wie Methyl, Ethyl, n-Propyl, iso-Propyl, n-Butyl, iso-Butyl, sec.-Butyl, tert.-Butyl und insbesondere Wasserstoff;
- R¹⁰, R¹¹: gleich oder vorzugsweise verschieden und gewählt aus C₁-C₂₇-Alkyl, wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, iso-Amyl, n-Hexyl, iso-Hexyl, sec.-Hexyl, n-Heptyl, iso-Heptyl, n-Octyl, n-Nonyl, n-Decyl, n-Undecyl, n-Dodecyl, n-Tridecyl, n-Tetradecyl, n-Hexadecyl, n-Octadecyl, n-Eicosyl; wobei die Summe der C-Atome von R¹⁰ und R¹¹ maximal 30 beträgt. Vorzugsweise hat R¹⁰ zwei C-Atome mehr als R¹¹; besonders bevorzugt sind beispielsweise die Kombinationen
R¹⁰ = n-Undecyl und R¹¹ = n-Nonyl,
R¹⁰ = n-Dodecyl und R¹¹ = n-Decyl,
R¹⁰ = n-Tridecyl und R¹¹ = n-Undecyl,
R¹⁰ = n-Tetradecyl und R¹¹ = n-Dodecyl,
R¹⁰ = n-Pentadecyl und R¹¹ = n-Tridecyl.
- R¹²: ist gewählt aus C₁-C₄-Alkyl wie Methyl, Ethyl, n-Propyl, iso-Propyl, n-Butyl, iso-Butyl, sec.-Butyl, tert.-Butyl,
Phenyl, ortho-Tolyl, meta-Tolyl, para-Tolyl
und insbesondere Wasserstoff.

In einer bevorzugten Ausführungsform der vorliegenden Erfindung ist genau einer der Reste R⁷ und R⁸ Wasserstoff und der andere Rest gewählt aus C₁-C₃₀-Alkyl.

In einer Ausführungsform der vorliegenden Erfindung sind alle oder zumindest ein gewisser Anteil, beispielsweise ein Drittel oder die Hälfte, der Sulfonylgruppen in als anionischem Emulgator eingesetzter Verbindung der allgemeinen Formel IV neutralisiert. Zur Neutralisation eignen sich beispielsweise basische Salze wie Hydroxide oder Carbonate der Alkalimetalle wie beispielsweise Na oder K. Zum Neutralisieren eignen sich weiterhin Ammoniak, Alkylamine wie beispielsweise Methylamin, Dimethylamin, Trimethylamin, Ethylamin, Diethylamin, Triethylamin, Ethylendiamin, und ganz besonders Alkanolamine wie beispielsweise Ethanolamin, Diethanolamin, Triethanolamin, N-Methyl-Ethanolamin, N-Methyldiethanolamin oder N-(n-Butyl)-diethanolamin.

Die Herstellung von Verbindungen der Formel IV ist an sich bekannt und in WO 01/68584 beschrieben. Sie gelingt beispielsweise durch einfache oder doppelte Veresterung von Dicarbonsäureanhydriden der allgemeinen Formel V 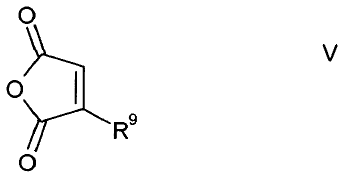 mit entsprechenden Alkoholen, die nicht rein vorliegen müssen, gefolgt von einer Umsetzung mit Disulfit.

Man kann anstatt einer reinen Verbindung der Formel IV Gemische von verschiedenen Schwefel-haltigen Verbindungen als Emulgator verwenden. Beispielsweise ist es möglich, zur Veresterung das als Oxoöl 135 oder Oxodicköl 135 (WO 01/68584) bekannte Gemisch einzusetzen.

In einer Ausführungsform der vorliegenden Erfindung können im erfindungsgemäßen Verfahren eingesetzte Formulierungen bis zu 40 Gew.-%, bevorzugt bis zu 20 Gew.-%, bezogen auf Emulgator, mindestens eines Alkohols der Formel VI 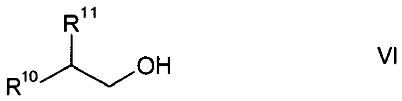 enthalten, wobei in Formel VI die Variablen R¹⁰ und R¹¹ wie oben stehend definiert sind.

Man kann das erfindungsgemäße Verfahren zur Herstellung von Leder vorzugsweise als Verfahren zum Nachgerben von Leder unter der Verwendung von Thioether durchführen. Das erfindungsgemäße Verfahren geht aus von konventionell gegerbten Halbzeugen, d.h. mit Chromgerbstoff alleine oder gegebenenfalls in Kombination mit mineralischen Gerbstoffen, Polymergerbstoffen, Aldehyden, Syntanen oder Harzgerbstoffen gegerbten Halbzeugen. Zur Durchführung des erfindungsgemäßen Verfahrens lässt man Thioether oder Verbindung der allgemeinen Formel III auf Halbzeuge einwirken.

Das erfindungsgemäße Verfahren kann man unter ansonsten üblichen Bedingungen der Nachgerbung durchführen. Man wählt zweckmäßig einen oder mehrere, d.h. beispielsweise 2 bis 6 Einwirkschritte und kann zwischen den Einwirkschritten mit Wasser spülen. Die Temperatur bei den einzelnen Einwirkschritten liegt jeweils im Bereich von 5 bis 60°C, bevorzugt 20 bis 45°C. Man setzt zweckmäßig weitere, während der Nachgerbung üblicherweise verwendete Mittel ein, beispielsweise Fettlicker, Nachgerbstoffe auf Basis von Harz- und Vegetabilgerbstoffen, Füllstoffe, Lederfarbstoffe oder Emulgatoren.

Durch das erfindungsgemäße Verfahren kann man Leder herstellen, die ausgezeichnete mechanische Eigenschaften wie beispielsweise Reißfestigkeiten und äußerst geringe Langzeitstabilität gegen die Vergilbung kombinieren und außerdem in Langzeittests eine sehr geringe Tendenz zur Bildung von Cr(V1) aufweisen. Ein Gegenstand der vorliegenden Erfindung sind demgemäß Leder, hergestellt nach dem erfindungsgemäßen Verfahren.

Die Langzeittests zur Ermittlung der Bildung von Cr(VI) kann man vorzugsweise bei 80°C nach EN ISO 17075:2007 durchführen.

Ein weiterer Aspekt der vorliegenden Erfindung ist die Verwendung der erfindungsgemäßen Leder zur Herstellung von Bekleidungsstücken, Möbeln oder Automobilinnenteilen. Unter Bekleidungsstücke sind im Sinne der vorliegenden Erfindung beispielsweise Jacken, Hosen, Schuhe, Gürtel oder Hosenträger zu nennen. Unter Möbeln sind im Zusammenhang mit der vorliegenden Erfindung alle solchen Möbel zu nennen, die Bestandteile aus Leder enthalten. Beispielhaft seien Sitzmöbel genannt wie etwa Sessel, Stühle, Sofas. Unter Automobilinnenteilen seien beispielhaft Autositze, Lenkradbespannungen und Bespannungen von Armaturenbrettern genannt.

Ein weiterer Aspekt der vorliegenden Erfindung sind Bekleidungsstücke, enthaltend die oder hergestellt aus erfindungsgemäßen Ledern. Ein weiterer Aspekt der vorliegenden Erfindung sind Möbel, enthaltend die oder hergestellt aus erfindungsgemäßen Ledern. Ein weiterer Aspekt der vorliegenden Erfindung sind Automobilinnenteile, enthaltend die oder hergestellt aus erfindungsgemäßen Ledern.

Die Erfindung wird durch Beispiele erläutert.

Es wurden die folgenden Thioether I.1, I.2 und II.1 eingesetzt:

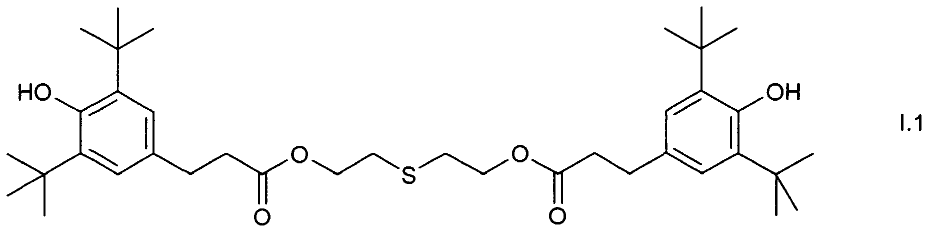

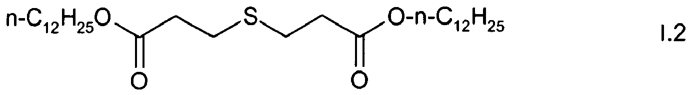

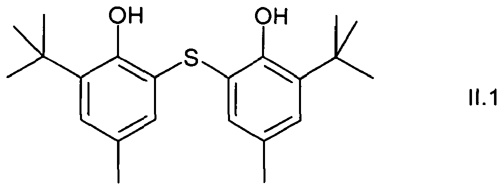

Als Vergleichssubstanzen wurden die oxidierbaren Verbindungen V-Phenol.1 bis V-Phenol.3 eingesetzt:

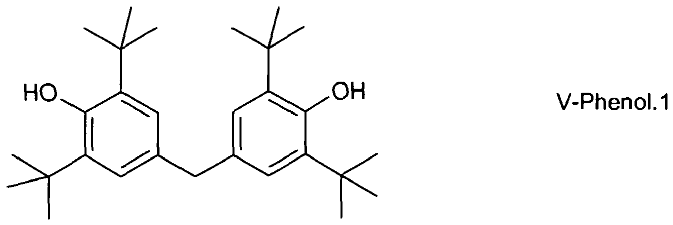

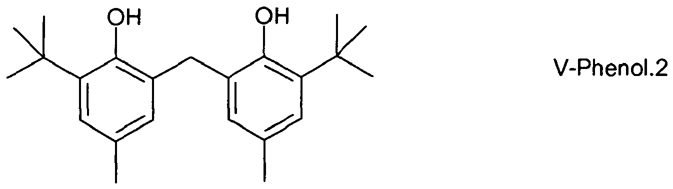

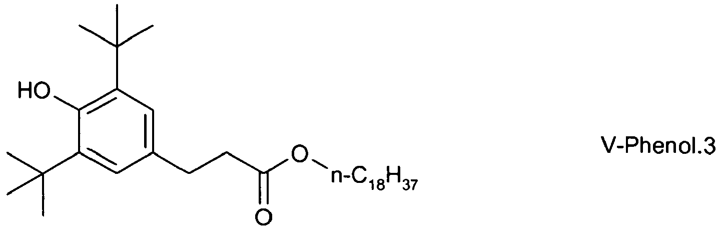

Thioether I.1, 1.2 und II.1, V-Phenol.1 bis V-Phenol.3 wurden nach Literaturvorschriften hergestellt bzw. sind kommerziell erhältlich.

In den Beispielen wurde kein Farbstoff verwendet, um die Vergilbung leichter messen zu können. Man kann die Beispiele jedoch auch durchführen unter Verwendung von einem oder mehreren Lederfarbstoffen.

Man behandelte erfindungsgemäß bzw. in Vergleichsversuchen nach der folgenden allgemeinen Vorschrift:
Angaben in % sind stets Gew.-% und beziehen sich auf das Falzgewicht. Die Angaben in % bzw. Gew.-% betreffen bei wässrigen Formulierungen stets den Feststoff- bzw. Wirkstoffanteil, wenn nicht ausdrücklich anders angegeben.

Ein Rinder-wet blue (US-Packer) wurde auf 2,2 mm gefalzt und auf den Kerbereich beschnitten. Anschließend wurde in einem Fass neutralisiert, indem man bei 20°C und einer Flottenlänge von 100 Gew.-% Wasser, 1 Gew.-% Natriumformiat und 0,5 Gew.-% Natriumbicarbonat über eine Zeit von 60 Minuten (unter Walken) einwirken ließ, bis ein pH-Wert von 5 bis 5,5 erreicht war. Anschließend wurden die wet blues zweimal mit je 100 % Wasser gewaschen und in acht Streifen zu je ca. 600 g geschnitten. Die Streifen wurden auf acht separate Fässer verteilt und wie folgt weiter behandelt.

Man beaufschlagte die Fässer mit je 100 % Wasser sowie mit 4% des Sulfongerbstoffs aus EP-B 0 459 168, Beispiel K1, versetzt. Nach einer Vorlaufzeit von 20 Minuten dosierte man in Folge 3 % einer 2,5 Gew.-% Thioether I.1, I.2 oder II.1, oder V-Phenol.1 bis V-Phenol.3-Lösung bzw. Dispersion in Triethylenglykol und danach jeweils 10 Gew.-% eines Fettlickers nach WO 03/023069, Beispiel A dosiert (Basis sulfitiertes Fischöl). Im achten Fass gab man weder Thioether noch Verbindung III.1 noch Vergleichssubstanz zu.

Anschließend walkte man 120 Minuten bei 30°C und säuerte mit 10% Ameisensäure auf einen pH-Wert der Flotte von 3,3 ab. Nach weiteren 20 Minuten ließ man die Flotte ab und wusch zweimal mit 100% Wasser. Die Leder werden über Nacht und mit einer Folie überdeckt auf Bock gelegt und dann bei 65°C für 4 Minuten im Vakuum getrocknet. Die Konditionierung vor den weiteren physikalisch-chemischen Tests erfolgte bei 40°C im Klimaraum über Nacht oder alternativ durch Einlegen in befeuchteten Sägespänen. Man erhielt die erfindungsgemäßen Leder L.1 bis L.3 und die Vergleichsleder V-L.4 bis V-L.8. Für die weiteren Tests wurden die Lederstreifen in entsprechend kleinere Stücke geteilt.

Die Fakra-Prüfung, eine Prüfung auf kurzfristige Vergilbungsstabilität, erfolgt gemäß DIN EN ISO 105-B06h bei 100°C unter Belichtung mit einer Hg-Lampe. Die Wärmevergilbung entsprechend Prüfverfahren bei 80°C wurde nach einem, drei, fünf und sieben Tagen in einem Notensystem von 1 bis 5 bewertet_ Jeher höher die Note, desto geringer die Neigung zur Vergilbung bzw. Wärmevergilbung. Note 5 bedeutet keine visuelle Verschlechterung/Vergilbung im Vergleich zur Lederprobe vor Beginn des Tests.

**Tabelle 1: Vergilbungstests an erfindungsgemäßen Lederproben und an Vergleichsleder**

| Beispiel | FAKRA | Wärmevergil bung 80°C/1d | Wärmevergil bung 80°C/3d | Wärmevergil bung 80°C/5d | Wärmevergil bung 80°C/7d |
|---|---|---|---|---|---|
| L.1 | 4 | 5 | 5 | 4 | 4 |
| L.2 | 4 | 3 | 2 | 2 | 1 |
| L.3 | 4 | 5 | 5 | 4 | 4 |
| V-L.5 | 2 | 2 | 2 | 1 | 1 |
| V-L.6 | 2 | 2 | 2 | 2 | 1 |
| V-L.7 | n.b. | 3 | 2 | 2 | 1 |
| V-L.8 | 2 | 2 | 2 | 1 | 1 |

| | | | | | |
|---|---|---|---|---|---|
| n.b.: nicht bestimmt | | | | | |

**Tabelle 2: Cr(VI)-Bestimmung an erfindungsgemäßen Lederproben und an Vergleichsleder**

| Beispiel | Cr(VI) nach 1d/80°C [ppm] | Cr(VI) nach 3d/80°C [ppm] | Cr(VI) nach 7d/80°C [ppm] |
|---|---|---|---|
| L.1 | <3 | <3 | <3 |
| L.2 | 9 | n.b. | n.b. |
| L.3 | <3 | <3 | 7 |
| V-L.5 | 14 | 26 | 49 |
| V-L.6 | 11 | 24 | 45 |
| V-L.7 | 12 | 19 | 31 |
| V-L.8 | 32 | 48 | 69 |

| | | | |
|---|---|---|---|
| n.b.: nicht bestimmt | | | |

Die Cr(VI)-Werte in ppm beziehen sich auf zerschnittenes Leder.

Aus erfindungsgemäßem Leder L.1 und L.3 lassen sich Schuhe Schuh.1 und Schuh.3 mit ausgezeichneten Gebrauchseigenschaften herstellen. Aus erfindungsgemäßem Leder L.2 lassen sich Schuhe Schuh.2 mit sehr guten Gebrauchseigenschaften herstellen. Die erfindungsgemäßen Schuhe Schuh.1 bis Schuh.3 zeigen äußerst geringe bzw. nicht messbare Neigung zum Vergilben. Auch sind die Cr(VI)-Gehalte der erfindungsgemäßen Schuhe Schuh.1 bis Schuh.3 nicht nachweisbar.

## Patentansprüche

1. Verfahren zur Herstellung von Leder, **dadurch gekennzeichnet, dass** man Cr(III)-haltige Halbzeuge in wässrigem Milieu mit einem oder mehreren Thioethern mit mindestens 10 C-Atomen pro Molekül behandelt.

2. Verfahren nach Anspruch 1, **dadurch gekennzeichnet, dass** der eingesetzte Thioether eine Löslichkeit in Wasser bei 20°C von höchstens 10 mg/l aufweist.

3. Verfahren nach Anspruch 1 oder 2, **dadurch gekennzeichnet, dass** eingesetzte Thioether gewählt sind aus Verbindungen der allgemeinen Formel I 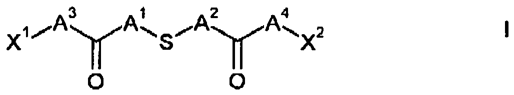 wobei X¹ und X² verschieden oder gleich sind und gewählt sind aus C₁₀-C₄₀-Alkyl, C₁₀-C₄₀-Alkoxy, C₆-C₁₄-Aryl, C₈-C₁₄-Hydroxyaryl, jeweils unsubstituiert oder einoder mehrfach substituiert mit C₁-C₄-Alkyl, und stickstoffhaltigen Heterocyclen, und wobei A¹, A², A³ und A⁴ verschieden sind oder gleich und gewählt aus Spacem der Formel (CHR¹)ₙ, wobei R¹ gewählt wird aus C₁-C₄-Alkyl oder Wasserstoff und n eine ganze Zahl im Bereich von null bis 10 ist.

4. Verfahren nach Anspruch 1 oder 2, **dadurch gekennzeichnet, dass** eingesetzte Thioether gewählt sind aus Verbindungen der allgemeinen Formel II 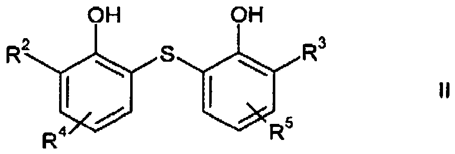 handelt, wobei die Variablen wie folgt definiert sind:
R², R³ gleich oder verschieden und gewählt aus iso-Amyl, tert.-Amyl, iso-Propyl, tert.-Butyl und sec.-Butyl,
R⁴, R⁵ gleich oder verschieden und gewählt aus Wasserstoff, Phenyl, Benzyl und C₁-C₄-Alkyl.

5. Verfahren nach einem der Ansprüche 1 bis 4, **dadurch gekennzeichnet, dass** die Behandlung in der Nachgerbung erfolgt.

6. Verfahren nach einem der Ansprüche 1 bis 5, **dadurch gekennzeichnet, dass** man gleichzeitig, vor oder nach der Behandlung mit Thioether mit mindestens 10 C-Atomen pro Molekül mit einem oder mehreren Fettungsmitteln behandelt.

7. Leder, hergestellt nach einem Verfahren nach einem der Ansprüche 1 bis 6.

8. Verwendung von Leder nach Anspruch 7 zur Herstellung von Möbeln, Schuhen oder Automobilinnenteilen.

9. Verfahren zur Herstellung von Möbeln, Schuhen oder Automobilinnenteilen unter Verwendung von Leder nach Anspruch 7.

## Claims

1. A process for the production of leather, wherein Cr(III)-containing semifinished products are treated in an aqueous medium with one or more thioethers having at least 10 carbon atoms per molecule.

2. The process according to claim 1, wherein the thioether used has a solubility of not more than 10 mg/l in water at 20°C.

3. The process according to claim 1 or 2, wherein thioethers used are selected from compounds of the general formula I 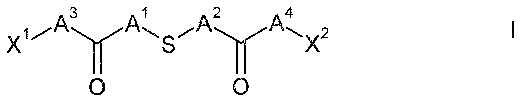 where X¹ and X² are different or identical and are selected from C₁₀-C₄₀-alkyl, C₁₀-C₄₀-alkoxy, C₆-C₁₄-aryl, C₆-C₁₄-hydroxyaryl, in each case unsubstituted or mono- or polysubstituted by C₁-C₄-alkyl, and nitrogen-containing heterocycles, and where A¹, A², A³ and A⁴ are different or identical and are selected from spacers of the formula (CHR¹)ₙ, where R¹ is selected from C₁-C₄-alkyl or hydrogen and n is an integer in the range from zero to 10.

4. The process according to claim 1 or 2, wherein thioethers used are selected from compounds of the general formula II 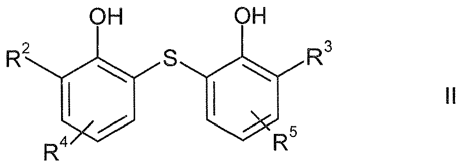 where the variables are defined as follows:
R², R³ are identical or different and are selected from isoamyl, tert-amyl, isopropyl, tert-butyl and sec-butyl,
R⁴, R⁵ are identical or different and are selected from hydrogen, phenyl, benzyl and C₁-C₄-alkyl.

5. The process according to any of claims 1 to 4, wherein the treatment is effected in the retanning.

6. The process according to any of claims 1 to 5, wherein treatment with one or more fatliquoring agents is effected simultaneously with, before or after the treatment with thioethers having at least 10 carbon atoms per molecule.

7. A leather produced by a process according to any of claims 1 to 6.

8. The use of leather according to claim 7 for the production of pieces of furniture, shoes or interior automotive parts.

9. A process for the production of pieces of furniture, shoes or interior automotive parts using leather according to claim 7.

## Revendications

1. Procédé de fabrication de cuir, **caractérisé en ce que** des produits semi-finis contenant Cr(III) sont traités dans un milieu aqueux avec un ou plusieurs thioéthers contenant au moins 10 atomes C par molécule.

2. Procédé selon la revendication 1, **caractérisé en ce que** le thioéther utilisé présente une solubilité dans l'eau à 20 °C d'au plus 10 mg/l.

3. Procédé selon la revendication 1 ou 2, **caractérisé en ce que** les thioéthers utilisés sont choisis parmi les composés de formule générale I 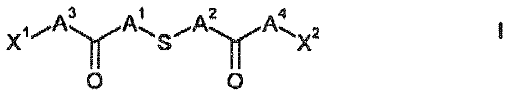 dans laquelle X¹ et X² sont identiques ou différents, et sont choisis parmi un alkyle en C₁₀-C₄₀, un alcoxy en C₁₀-C₄₀, un aryle en C₆-C₁₄, un hydroxyaryle en C₆-C₁₄, chacun non substitué ou substitué une ou plusieurs fois avec un alkyle en C₁-C₄, et les hétérocycles contenant de l'azote, et dans laquelle A¹, A², A³ et A⁴ sont identiques ou différents, et choisis parmi les espaceurs de formule (CHR¹)ₙ, R¹ étant choisi parmi un alkyle en C₁-C₄ ou l'hydrogène et n étant un nombre entier dans la plage allant de zéro à 10.

4. Procédé selon la revendication 1 ou 2, **caractérisé en ce que** les thioéthers utilisés sont choisis parmi les composés de formule générale II 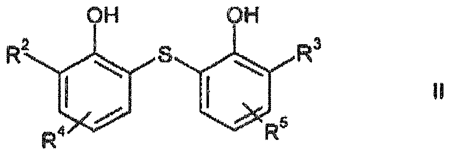 dans laquelle les variables sont définies de la manière suivant
R², R³ identiques ou différents, et choisis parmi iso-amyle, tert.-amyle, iso-propyle, tert.-butyle et sec.-butyle,
R⁴, R⁵ identiques ou différents, et choisis parmi l'hydrogène, phényle, benzyle et alkyle en C₁-C₄.

5. Procédé selon l'une quelconque des revendications 1 à 4, **caractérisé en ce que** le traitement a lieu pendant le retannage.

6. Procédé selon l'une quelconque des revendications 1 à 5, **caractérisé en ce qu'**un traitement avec un ou plusieurs agents graissants est réalisé simultanément, avant ou après le traitement avec un thioéther contenant au moins 10 atomes C par molécule.

7. Cuir, fabriqué par un procédé selon l'une quelconque des revendications 1 à 6.

8. Utilisation d'un cuir selon la revendication 7 pour la fabrication de meubles, de chaussures ou de parties intérieures d'automobiles.

9. Procédé de fabrication de meubles, de chaussures ou de parties intérieures d'automobiles utilisant un cuir selon la revendication 7.
